# FASCICULE DE BREVET EUROPEEN

(11) **EP 1 510 189 B1**
(45) Date de publication et mention de la délivrance du brevet: **30.04.2008**
(21) Numéro de dépôt: 04292068.6
(22) Date de dépôt: 20.08.2004
(51) Int. Cl.: A61F 2/12

(54) **Coque mammaire**
Brustschale
Female breast cage

(30) Priorité: 29.08.2003 FR 0310298; 20.11.2003 FR 0313613
(43) Date de publication de la demande: 02.03.2005
(73) Titulaire: Perouse Plastie, 60540 Bornel (FR)
(72) Inventeur: Barreau-Pouhaer, Lise, 92160 Antony (FR)
(74) Mandataire: Blot, Philippe Robert Emile

(56) Documents cités:
- FR-A- 2 682 284
- GB-A- 2 151 927
- US-A- 4 157 085
- US-A- 4 597 763
- US-A1- 2001 041 936
- US-B1- 6 210 439

## Description

La présente invention concerne une coque mammaire, du type comportant une paroi en matériau polymère déformable élastiquement.

En chirurgie plastique ou reconstructive, les chirurgiens sont amenés à implanter des prothèses mammaires.

Les prothèses connues comportent une poche souple fermée faite en silicone à l'intérieur de laquelle est confiné un liquide de remplissage tel que du sérum physiologique ou du gel de silicone. Le fluide de remplissage garantit un aspect visuel et un touché de la prothèse proche de la glande mammaire initial.

Cependant, certaines prothèses présentent des risques de ruptures qui peuvent conduire à une libération du fluide de remplissage dans l'organisme, ce qui n'est pas souhaitable. De plus, un suintement de ce fluide au travers de l'enveloppe peut arriver en vieillissant.

De plus, il est fréquent que lors de l'intervention, et avant la mise en place de la prothèse mammaire définitive, les chirurgiens utilisent des gabarits, également dénommés « simulateurs » ou « fantômes » afin d'évaluer le résultat esthétique final avant la pose de la prothèse définitive. Ces gabarits sont implantés temporairement puis retirés pour permettre la mise en place de la prothèse définitive.

Les gabarits ont un volume identique à celui de la prothèse définitive.

Ainsi, le chirurgien peut apprécier l'adéquation entre la patiente et la prothèse mammaire définitive avant même que celle-ci soit mise en place.

Il arrive parfois que plusieurs essais de gabarits de tailles différentes soient nécessaires pour atteindre un résultat satisfaisant.

On connaît des gabarits qui sont en fait de simples prothèses mammaires constituées d'une enveloppe souple clause contenant un matériau de remplissage tel qu'un gel ou du sérum physiologique. Les gabarits sont généralement des prothèses déclassées, qui sont colorées pour attirer l'oeil du chirurgien et ainsi éviter qu'elle soit confondue avec la prothèse définitive.

Ces gabarits issus de prothèse mammaire présentent l'inconvénient d'être coûteux. Ils sont donc utilisés plusieurs fois après lavage et re-stérilisation. Or, le processus de re-stérilisation détériore considérablement la qualité du gel contenu dans la prothèse. Ces stérilisations et utilisations successives présentent donc un risque non admissible pour la patiente.

Par ailleurs, US. 6,210,439 décrit une coque selon le préambule de la revendication 1.

L'invention a pour but de proposer un matériel fiable et peu coûteux à l'usage pour les interventions de chirurgie mammaire, esthétique ou reconstructive.

A cet effet, l'invention a pour objet une coque selon la revendication 1.

Suivant des modes particuliers de réalisation, la coque comporte l'une ou plusieurs des caractéristiques des revendications dépendantes.

L'invention sera mieux comprise à la lecture de la description qui va suivre, donnée uniquement à titre d'exemple et faite en se référant aux dessins, sur lesquels :
- la figure 1 est une vue en perspective d'un premier mode de réalisation d'une coque selon l'invention ;
- la figure 2 est une vue en section de la coque de la figure 1 ;
- la figure 2A est une vue identique à celle de la figure 2 d'une variante de réalisation de la coque des figures 1 et 2 ;
- la figure 3 est une vue en section d'une variante de réalisation de la coque illustrée aux figures 1 et 2 ; et
- la figure 4 est une vue en perspective d'un autre mode de réalisation de la coque selon l'invention.

Les coques 10 illustrées aux figures sont destinées à former indifféremment des prothèses mammaires implantables ou des gabarits de prothèse mammaire. C'est pourquoi, elles seront désignées dans la suite par le terme générique "coque".

Une prothèse mammaire implantable est destinée à être implantée définitivement par un chirurgien soit sous le muscle pectoral, soit directement sous la glande mammaire.

Au contraire, un gabarit de prothèse mammaire est destiné à être placé temporairement par un chirurgien soit sous le muscle pectoral, soit directement sous la glande mammaire puis à être extrait et remplacé par une prothèse mammaire définitive qui peut ou non être de même structure.

La coque 10 présente une forme générale de cloche. Plus précisément, elle a une forme hémisphérique. Ainsi, elle présente extérieurement une surface convexe 12 et une surface intérieure concave 14 délimitant un évidement 16 dépourvu de tout remplissage.

Ainsi, la coque 10 est formée par une paroi 17 en matériau polymère d'une épaisseur constante comprise entre 0,5 et 10 mm et de préférence entre 2 et 5 mm. Cette paroi présente un bord périphérique d'appui 18 délimitant l'ouverture par laquelle débouche l'évidement 16. Le bord 18 s'étend sensiblement dans un même plan. Depuis le bord 18, la coque présente une section, prise parallèlement au plan du bord 18 qui est progressivement décroissante du bord 18 vers le sommet noté 20 de la coque.

La hauteur de la coque, mesurée entre sa base 18 et le sommet 20 est comprise entre 15 et 100 mm.

Le diamètre de la base 18 est compris entre 70 et 200 mm.

La structure de la coque est telle que celle-ci puisse être élastiquement déformée, par écrasement de son bord ouvert de sorte que le bord 18 puisse être aplati sur lui-même.

En variante, la coque est roulée autour du bord 18.

La structure de la coque est suffisamment souple et élastique pour que la déformation de la coque, conduisant à la disparition de l'évidement 16 par rapprochement des parties opposées de la paroi 17 puisse être réalisée manuellement. Dans cette position repliée, la coque peut être introduite sous la peau de la patiente.

En variante, la coque est enroulée autour du bord 18.

En outre, la matière est suffisamment élastique et la structure est telle que la coque reprend sa forme lorsqu'elle est relâchée sous la seule action de l'élasticité de la paroi 17, même lorsqu'elle est contrainte par la pression appliquée par la peau, et éventuellement la glande mammaire préexistante de la patiente.

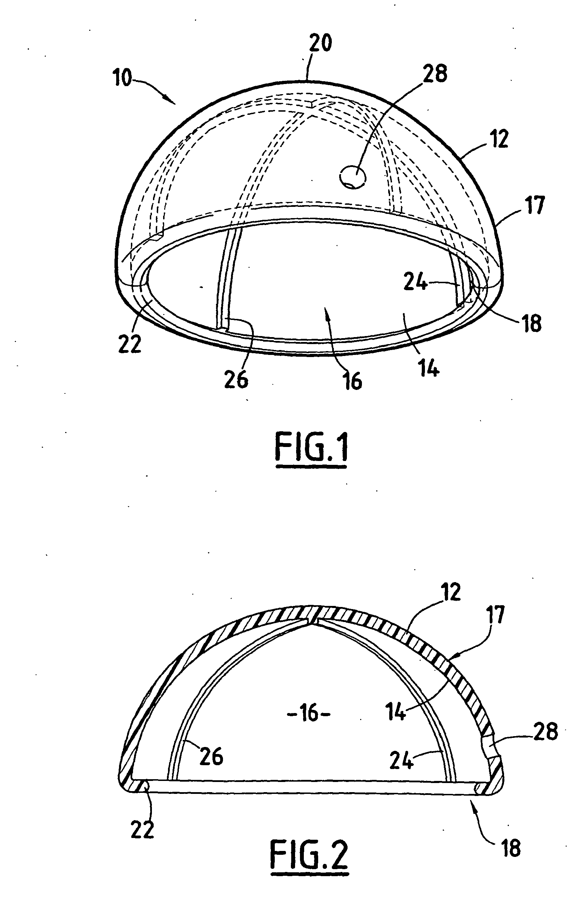

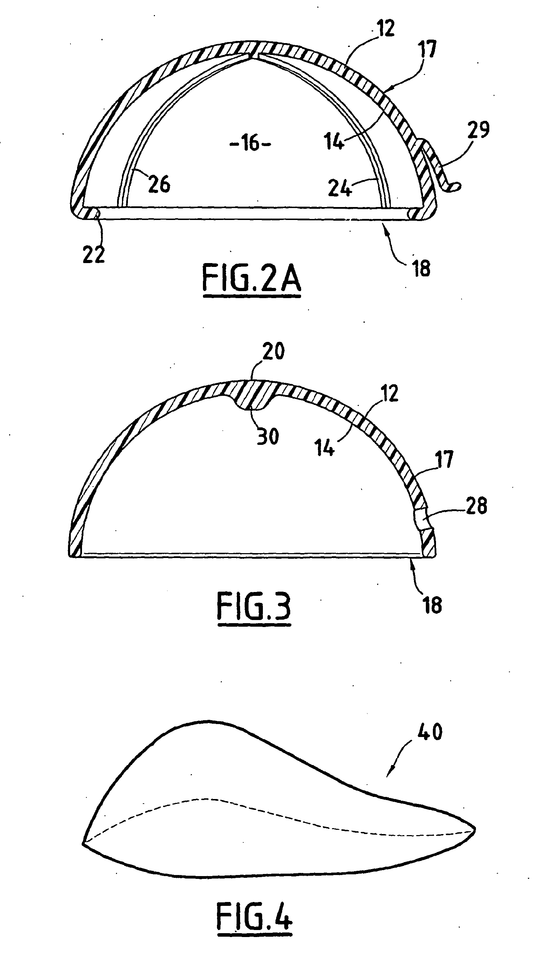

Afin d'assurer une rigidification de la coque, le bord 18 présente avantageusement un rebord périphérique rentrant 22 mieux visible sur la figure 2. Ce rebord est venu de matière avec le reste de la paroi 17. Il forme une nervure et présente une épaisseur sensiblement égale à l'épaisseur moyenne de la paroi 17. Il s'étend radialement sur une distance inférieure au rayon de la base, de façon à ce que celle-ci reste ouverte. Cette distance est notamment comprise entre 1 mm et le rayon de la base diminué de 5 mm.

Avantageusement, le rebord 22 est légèrement tronconique et est incurvé vers l'intérieur de la coque. Ainsi, il définit une surface d'appui concave propre à épouser exactement la surface de l'organisme sur laquelle il s'applique après implantation.

Afin d'augmenter également la rigidification de la coque, celle-ci comporte avantageusement sur la surface intérieure 14, deux nervures de rigidification semi-équatoriales 24, 26 décalées l'une par rapport à l'autre d'environ 90° et se croisant au voisinage du sommet 20 de la coque.

Ses nervures s'étendent depuis deux points diamétralement opposés du bord 18. Elles ont une hauteur comprise entre 1 et 5 mm.

De préférence, les nervures 22, 24 et 26 sont venues de matière avec la paroi 17. Toutefois, elles peuvent être formées dans un autre matériau de sorte que la coque est bi-matière.

Des raidisseurs semi-équatoriaux formés par exemple de tiges métalliques déformables sont, en variante, intégrés dans la paroi 17.

Par ailleurs, avantageusement, la coque présente, dans sa paroi 17, un profil de préhension permettant au chirurgien de la saisir facilement pour son retrait de la loge mammaire.

Dans le mode de réalisation des figures 1 et 2, ce profil de préhension est formé d'une lumière 28 traversant la paroi 17 de part en part. Cette lumière présente un diamètre suffisant pour l'introduction d'un organe de récupération. Ainsi, la lumière 28 est formée, par exemple, d'un trou circulaire traversant ayant un diamètre supérieur à 8 mm. Ce diamètre est de préférence compris entre 8 mm et 20 mm.

Dans la variante de réalisation illustrée sur la figure 2A, la lumière 28 est remplacée par une patte de préhension 29 faisant saillie sur la surface extérieure de la paroi 17. Cette patte de préhension forme une saillie facilement saisissable par le praticien lorsque la coque est dans la loge mammaire. Cette patte est venue de matière avec la paroi 17 ou ajoutée. Elle est formée de préférence dans l'un des matériaux mentionnés pour la paroi 17.

Une telle coque est formée par moulage, trempage, coulée ou injection de polymères.

Dans le cas d'une prothèse mammaire implantable, le matériau utilisé pour former la paroi 17 et tous les autres éléments éventuels de la prothèse est un matériau répondant aux exigences réglementaires propres aux prothèses mammaires implantables. Le matériau est en particulier adapté pour être maintenu pendant au moins dix ans dans l'organisme.

Qu'il s'agisse d'une prothèse ou d'un gabarit, le matériau pour former la coque est par exemple du polyéthylène basse densité, du polychlorure de vinyle (PVC), du polyuréthane, du silicone, ou tout autre polymère semi-rigide. Ce polymère est stérilisable de manière à ce que lors de sa commercialisation, la coque soit stérile et emballée dans un conditionnement étanche. Ainsi, avant toute intervention, la coque est fournie stérile et emballée dans son conditionnement étanche.

On comprend que la forme en cloche de la coque fait que celle-ci est formée de peu de matière et est donc d'un coût extrêmement réduit.

En outre, l'absence de matière dans l'évidement 16, permet que la coque puisse être facilement pliée en vue de son introduction sous la peau de la patiente. L'élasticité propre à la forme en cloche de la coque permet un déploiement aisé de celle-ci après introduction sous la peau.

Enfin, la coque n'étant formée que par une paroi déformable, celle-ci ne comporte pas de gel ou tout autre liquide de remplissage, ce qui supprime les risques de suintements et de libérations involontaires de fluide dans l'organisme, notamment dans le cas d'une prothèse.

Sur la figure 3 est représenté schématiquement un autre mode de réalisation de la coque. Pour faciliter son déploiement, la paroi 17 présente dans la région du sommet 20 une excroissance interne 30 faisant saillie à la surface intérieure 14 de la coque. Cette excroissance 30 est par exemple hémisphérique et présente une hauteur comprise entre 1 et 10 mm.

Dans encore une autre variante illustrée sur la figure 4, la coque 40 présente non pas une forme hémisphérique mais une forme dite anatomique, c'est-à-dire que le sommet de la coque est excentré par rapport à la base 18, laquelle est de plus généralement allongée.

Ainsi, la coque présente une forme de goutte qui peut être dissymétrique par rapport à son axe vertical.

Dans le cas où la coque constitue un gabarit, celle-ci peut être utilisée par la patiente, sur la peau, de façon à apprécier le volume choisi, lors d'une consultation pré-opératoire.

## Revendications

1. Coque mammaire, comportant une paroi (17), laquelle paroi (17) en position déployée présente une forme de cloche présentant un sommet (20) et ladite paroi (17) délimite un évidement interne (16) débouchant à la base de la coque suivant une ouverture délimitée par un bord (18) d'appui de la coque opposé audit sommet (20), **caractérisée en ce que** ladite paroi est en matériau polymère déformable élastiquement, et **en ce qu'**elle est adaptée pour un déploiement autonome depuis une position repliée à sa position déployée sous l'action de la seule élasticité de la paroi (17).

2. Coque suivant la revendication 1, **caractérisée en ce que** le bord d'appui (18) s'étend généralement dans un même plan.

3. Coque selon l'une quelconque des revendications précédentes, **caractérisée en ce qu**'elle comporte un rebord périphérique intérieur (22) s'étendant suivant le bord d'appui (18) à la périphérie de l'ouverture.

4. Coque selon la revendication 3, **caractérisée en ce qu**e le rebord périphérique (22) présente globalement un profil tronconique délimitant une surface d'appui concave de la coque.

5. Coque selon l'une quelconque des revendications précédentes, **caractérisée en ce qu**'elle comporte au moins un raidisseur semi-équatorial (24, 26) s'étendant entre deux points opposés du bord (18), le ou chaque raidisseur semi-équatorial (24, 26) passant sensiblement par le sommet (20) de la coque.

6. Coque selon la revendication 5, **caractérisée en ce que** le ou chaque raidisseur est une nervure faisant saillie sur la surface intérieure (14) de la paroi (17).

7. Coque selon l'une quelconque des revendications précédentes, **caractérisée en ce qu'**elle comporte une protubérance intérieure (30) au voisinage du sommet (20) de la coque, laquelle protubérance fait saillie par rapport à la surface intérieure (14) de la paroi (17).

8. Coque selon l'une quelconque des revendications précédentes, **caractérisée en ce que** ladite paroi (17) est comprise entre 0,5 mm et 10 mm et de préférence entre 2 mm et 5 mm.

9. Coque selon l'une quelconque des revendications précédentes, **caractérisée en ce que** la paroi (17) est formée d'un matériau choisi dans le groupe consistant en du polyéthylène basse densité, du polychlorure de vinyle, du polyuréthane et du silicone.

10. Coque selon l'une quelconque des revendications précédentes, **caractérisée en ce que** la base de la coque délimitée par le bord d'appui (18) a une dimension moyenne comprise entre 70 mm et 200 mm, la hauteur de la coque mesurée entre le sommet (20) et la base étant comprise entre 15 mm et 100 mm.

11. Coque selon l'une quelconque des revendications précédentes, **caractérisée en ce que** la paroi (17) est formée d'un matériau pour prothèses mammaires implantables.

12. Coque selon l'une quelconque des revendications précédentes, **caractérisée en ce qu**'elle comporte, sur la paroi (17), un profil de préhension (28 ; 29).

13. Coque selon l'une quelconque des revendications précédentes, **caractérisée en ce qu**'elle constitue une prothèse mammaire implantable (10) et qu'elle est formée de matériaux pour prothèses mammaires implantables.

14. Coque selon l'une quelconque des revendications 1 à 12, **caractérisée en ce qu**'elle constitue un gabarit de prothèse mammaire (10) et qu'elle est formée de matériaux pour gabarits de prothèses mammaires implantables.

## Claims

1. Mammary shell, consisting of a wall (17), where the wall (17) in the deployed position takes the form of a dome which possesses a summit (20) and where the said wall (17) surrounds an internal cavity (16) which opens out at the base of the shell in the form of an opening bounded by a support rim (18) for the shell opposite the said summit (20), **characterised by** the fact that the said wall is made of elastically deformable polymer material, and by the fact that it is suitable for autonomous deployment from a folded position to its deployed position solely due to the action of the elasticity of the wall (17).

2. Shell according to claim 1, **characterised by** the fact that the support rim (18) generally extends in the same plane.

3. Shell according to any of the preceding claims whatsoever, **characterised by** the fact that it includes an internal peripheral edge (22) extending along the support rim (18) to the periphery of the opening.

4. Shell according to claim 3, **characterised by** the fact that the peripheral edge (22) overall has a truncated conical section bounding a concave support surface for the shell.

5. Shell according to any of the preceding claims whatsoever, **characterised by** the fact that it includes at least one semi-equatorial stiffener (24, 26) which extends between two opposite points of the rim (18), where the (or each) semi-equatorial stiffener (24, 26) effectively passes through the summit of the shell.

6. Shell according to claim 5, **characterised by** the fact that the (or each) stiffener is a protruding rib on the internal surface (14) of the wall (17).

7. Shell according to any of the preceding claims whatsoever, **characterised by** the fact that it includes an internal protuberance (30) close to the summit (20) of the shell, and that the said protuberance protrudes in relation to the internal surface (14) of the wall (17).

8. Shell according to any of the preceding claims whatsoever, **characterised by** the fact that the said wall (17) is between 0.5 mm and 10 mm, and preferably between 2 mm and 5 mm.

9. Shell according to any of the preceding claims whatsoever, **characterised by** the fact that the wall (17) is formed of a material selected from a group made up of low-density polyethylene, polyvinylchloride, polyurethane and silicone.

10. Shell according to any of the preceding claims whatsoever, **characterised by** the fact that the base of the shell bounded by the support rim (18) has a mean dimension of between 70 mm and 200 m, with the height of the shell measured between the summit (20) and the base being between 15 mm and 100 mm.

11. Shell according to any of the preceding claims whatsoever, **characterised by** the fact that the wall (17) is formed of a material for implantable breast prostheses.

12. Shell according to any of the preceding claims whatsoever, **characterised by** the fact that it includes, on the wall (17), a gripping profile (28; 29).

13. Shell according to any of the preceding claims whatsoever, **characterised by** the fact that it forms an implantable breast prosthesis (10) and that it is formed of materials for implantable breast prostheses.

14. Shell according to any of the preceding claims 1 to 12 whatsoever, **characterised by** the fact that it forms a pattern for an implantable breast prosthesis (10) and that it is formed of materials for patterns for implantable breast prostheses.

## Patentansprüche

1. Brustschale, die eine Wand (17) aufweist, wobei die Wand (17) in einer entfalteten Position eine Glockenform aufweist, die einen Scheitelpunkt (20) aufweist, und wobei die Wand (17) eine innere Aussparung (16) begrenzt, die an der Basis der Schale entlang einer Öffnung ausmündet, die durch einen Stützrand (18) der Schale gegenüber dem Scheitelpunkt (20) begrenzt ist, **dadurch gekennzeichnet, dass** die Wand aus einem elastisch verformbaren Polymer-Material ist, sowie **dadurch**, dass sie für ein eigenständiges Entfalten lediglich durch die Wirkung der Elastizität der Wand (17) aus einer zusammengefalteten Position in ihre entfaltete Position angepasst ist.

2. Schale gemäß Anspruch 1, **dadurch gekennzeichnet, dass** sich der Stützrand (18) im Allgemeinen in einer gleichen Ebene erstreckt.

3. Schale gemäß einem der vorherigen Ansprüche, **dadurch gekennzeichnet, dass** sie einen inneren Umfangsrand (22) aufweist, der sich entlang dem Stützrand (18) an dem Umfang der Öffnung erstreckt.

4. Schale gemäß Anspruch 3, **dadurch gekennzeichnet, dass** der Umfangsrand (22) insgesamt ein kegelförmiges Profil aufweist, das eine konkave Stützfläche der Schale begrenzt.

5. Schale gemäß einem der vorherigen Ansprüche, **dadurch gekennzeichnet, dass** sie mindestens eine halb-äquatoriale Versteifung (24, 26) aufweist, die sich zwischen zwei gegenüberliegenden Punkten des Randes (18) erstreckt, wobei die oder jede halb-äquatoriale Versteifung (24, 26) im Wesentlichen durch den Scheitelpunkt der Schale verläuft.

6. Schale gemäß Anspruch 5, **dadurch gekennzeichnet, dass** die oder jede Versteifung eine Rippe ist, die an der Innenfläche (14) der Wand (17) hervorsteht.

7. Schale gemäß einem der vorherigen Ansprüche, **dadurch gekennzeichnet, dass** sie eine innere Ausstülpung (30) in der Nähe des Scheitelpunktes (20) der Schale aufweist, wobei die Ausstülpung relativ zu der Innenfläche (14) der Wand (17) hervorsteht.

8. Schale gemäß einem der vorherigen Ansprüche, **dadurch gekennzeichnet, dass** die Wand (17) zwischen 0,5 mm und 10 mm und vorzugsweise zwischen 2 mm und 5 mm beträgt.

9. Schale gemäß einem der vorherigen Ansprüche, **dadurch gekennzeichnet, dass** die Wand (17) aus einem Material gebildet ist, das aus einer Gruppe ausgewählt ist, die aus Polyethylen mit niedriger Dichte, Polyvinylchlorid, Polyurethan und Silikon besteht.

10. Schale gemäß einem der vorherigen Ansprüche, **dadurch gekennzeichnet, dass** die Basis der Schale, die durch den Stützrand (18) begrenzt ist, eine mittlere Abmessung zwischen 70 mm und 200 mm aufweist, wobei die Höhe der Schale, gemessen zwischen dem Scheitelpunkt (20) und der Basis zwischen 15 mm und 100 mm liegt.

11. Schale gemäß einem der vorherigen Ansprüche, **dadurch gekennzeichnet, dass** die Wand (17) aus einem Material für implantierbare Brustprothesen gebildet ist.

12. Schale gemäß einem der vorherigen Ansprüche, **dadurch gekennzeichnet, dass** sie an der Wand (17) ein Greifprofil (28, 29) aufweist.

13. Schale gemäß einem der vorherigen Ansprüche, **dadurch gekennzeichnet, dass** sie eine implantierbare Brustprothese (10) bildet, und **dadurch**, dass sie aus Materialien für implantierbare Brustprothesen gebildet ist.

14. Schale gemäß einem der Ansprüche 1 bis 12, **dadurch gekennzeichnet, dass** sie eine Schablone für eine Brustprothese (10) bildet, und **dadurch**, dass sie aus Materialien für Schablonen für implantierbare Brustprothesen gebildet ist.
